# EUROPEAN PATENT APPLICATION

(11) **EP 3 421 032 A1**
(43) Date of publication of application: **02.01.2019**
(21) Application number: 17756490.3
(22) Date of filing: 21.02.2017
(51) Int. Cl.: A61K 9/14, A61K 9/20, A61K 31/135, A61K 31/136, A61K 31/445, A61K 47/10, A61K 47/32, A61K 47/38

(54) **PHARMACEUTICAL COMPOSITION PARTICLES, ORALLY DISINTEGRATING PREPARATION INCLUDING SAME, AND METHOD FOR PRODUCING PHARMACEUTICAL COMPOSITION PARTICLES**

(30) Priority: 23.02.2016 JP 2016031860
(71) Applicant: Nipro Corporation, Osaka-shi, Osaka 531-8510 (JP); Zensei Pharmaceutical Industries Co., Ltd., Sakai-shi, Osaka 593-8307 (JP)
(72) Inventor: HAYASHIDA, Tomohiro, Kita-ku Osaka-shi, Osaka 5318510 (JP); HOASHI, Yohei, Kita-ku Osaka-shi, Osaka 5318510 (JP); IJITSU, Shin, Kita-ku Osaka-shi, Osaka 5318510 (JP); NAKANO, Yoshio, Kishiwada-shi Osaka 596-0808 (JP); YAMAZAKI, Junji, Kishiwada-shi Osaka 596-0808 (JP); INOUE, Katsuhisa, Kishiwada-shi Osaka 596-0808 (JP); AIZAWA, Atsushi, Kishiwada-shi Osaka 596-0808 (JP)
(74) Representative: Reitstötter Kinzebach
(86) International application number: PCT/JP2017/006388
(87) International publication number: WO 2017/146052

(57) **Abstract**

Provided are pharmaceutical composition particles which are capable of achieving both of masking of an unpalatable taste and improvement in dissolution properties; an orally disintegrating tablet including the pharmaceutical composition particles; and a method for manufacturing the pharmaceutical composition particles. Each of the pharmaceutical composition particles includes: a drug-containing core particle; an intermediate layer containing a gelling swelling substance and coating an outside of the core particle; and an outermost layer containing a water-insoluble substance and coating an outside of the intermediate layer. In addition, the method for manufacturing the pharmaceutical composition particles includes: a pulverization step of pulverizing the gelling swelling substance so as to have an average particle diameter of 15 µm or less; a suspension step of obtaining a suspension by suspending the gelling swelling substance pulverized at the pulverization step in an organic solvent; an intermediate layer formation step of forming an intermediate layer on an outside of a core particle by spraying the suspension obtained at the suspension step onto the core particle containing a drug; and an outer layer formation step of forming an outer layer by coating an outside of the intermediate layer with a water-insoluble substance.

## Description

### TECHNICAL FIELD

The present invention relates generally to pharmaceutical composition particles for oral administration, an orally disintegrating tablet including the pharmaceutical composition particles, and a method for manufacturing the pharmaceutical composition particles; and in particular, the present invention relates to pharmaceutical composition particles for oral administration, which are release-controllable for masking of an unpalatable taste and improvement in dissolution properties, an orally disintegrating tablet including the pharmaceutical composition particles, and a method for manufacturing the pharmaceutical composition particles.

### BACKGROUND ART

Dosage forms of oral pharmaceutical composition particles such as granules, fine granules, and powder have sizes smaller than those of dosage forms of a tablet and a capsule. These dosage forms of the oral pharmaceutical composition particles allow even patients having difficulties in swallowing the tablet and the capsule to easily take drugs of the pharmaceutical composition particles. In recent years, dosage forms such as the above-mentioned dosage forms which enhance convenience for patients as well as hospitals have been attracting attention. Among the drugs in the dosage forms having the high convenience, an orally disintegrating tablet has high convenience, which allows administration without water and easy swallowing and has suitability for tube administration. Therefore, a variety of ingenuity has been exercised for a method for manufacturing fine particles which form the orally disintegrating tablet.

The pharmaceutical composition particles, such as fine particles having an average particle diameter of 400 µm or less in particular, have a large surface area per weight, as compared with fine particles having a larger average particle diameter than the above-mentioned average particle diameter. In other words, the fine particles having the small size have a larger area in contact with water in the oral cavity than comparatively large fine particles and are rapid in a speed of infiltration of water into the particles. Therefore, when the pharmaceutical composition particles are taken, a tendency for the drug to be rapidly released in the oral cavity is strong. There may be a case where this causes a variety of problems. For example, there may be a case where when a drug has an unpalatable taste, the drug rapidly released in the oral cavity gives a strong feeling of discomfort to a patient, thus markedly lowering administration compliance. In addition, when a drug to be absorbed within the oral cavity is included in the pharmaceutical composition particles, the drug rapidly released in the oral cavity may cause problems such as side effects and development of differences in drug efficacy among individuals.

In order not to cause these problems, it is required to suppress drug release of the pharmaceutical composition particles in the oral cavity for a predetermined time. By suppressing the drug release for the predetermined time during which the pharmaceutical composition particles are present within the oral cavity, the unpalatable taste can be masked. In addition, the problems such as the side effects and the development of differences in drug efficacy among individuals can also be avoided.

On the other hand, in order for a drug to develop sufficient drug efficacy, it is required for the drug to be released from oral pharmaceutical composition particles and for a sufficient quantity thereof to be absorbed into the body. The orally taken preparation moves through a gastrointestinal tract over time. In general, it is often the case that the drug is absorbed in an upper gastrointestinal tract.

Taking these into consideration, after suppressing the drug release from the oral pharmaceutical composition particles for a predetermined time, it is desired that the pharmaceutical composition particles are disintegrated as rapidly as possible and the drug is rapidly released and absorbed in the upper gastrointestinal tract.

Accordingly, in order to achieve the purposes such as masking of the unpalatable taste of a drug and avoiding of absorption thereof within the oral cavity, it is required to suppress the drug release in the oral cavity for a predetermined time. At the same time, it is crucial that the drug is rapidly released from the oral pharmaceutical composition particles in the gastrointestinal tract. In addition, a degree of the unpalatable taste, duration of the unpalatable taste, and an absorption speed in the oral cavity vary among drugs. In designing the pharmaceutical composition particles, it is extremely important that in accordance with properties of a drug included in the pharmaceutical composition particles, an optimum combination of the time at which the drug release starts in the oral cavity and a drug release time after orally taking the drug is achieved.

In order to realize the above-mentioned optimum combination, it is required to design pharmaceutical composition particles which allow a time, during which the initial drug release is suppressed (hereinafter, also referred to as a "lag time"), to be arbitrarily controlled in accordance with properties of a drug or a preparation and which after orally taking the drug, release the drug after the lapse of a predetermined time at a targeted speed. In other words, a technology for freely controlling the combination of suppressing of the drug release time in the oral cavity and the drug release speed in a living body has been demanded.

In the meantime, the orally disintegrating tablet attracting the attention from the point of view of the convenience is devised to contain pharmaceutical composition particles whose drug release is controlled for purposes such as masking of the unpalatable taste. In order to reduce a rough feeling of the pharmaceutical composition particles contained in the orally disintegrating tablet in the oral cavity, it is required that a size of each thereof is further made smaller than a size of each of the oral pharmaceutical composition particles of granules, fine granules, powder, or the like, and specifically, is made to have an average particle diameter of 400 µm or less and preferably, to have an average particle diameter of 300 µm or less. However, in accordance with a decrease in the size of each of the pharmaceutical composition particles, the tendency for the drug to be unnecessarily rapidly released becomes strong. Therefore, in order to freely control the combination of suppressing of the drug release time in the oral cavity and the drug release speed in the living body, ingenuity for highly advanced formulation is demanded. In reality, it has been extremely difficult for the general formulation method to concurrently satisfy "the requirement that is suppressing of the initial drug release of the above-mentioned minute oral pharmaceutical composition particles (controlling of the lag time)" and "the requirement that is the subsequent rapid drug release".

Ordinarily, in order to mask the unpalatable taste by controlling the drug release of the oral pharmaceutical composition particles, a method for coating the particles with various kinds of film forming materials is employed. For example, when the oral pharmaceutical composition particles which contain a drug having the unpalatable taste are coated with a water-insoluble polymer, infiltration of water into an inside of each of the particles is suppressed, whereby the release of the drug from the inside of the particles is suppressed and masking of the unpalatable taste is achieved.

In the above-mentioned method, even when the water infiltrates into the particles, the film formed of the water-insoluble polymer does not break down and a drug release speed remains suppressed. Therefore, the rapid drug release after the lag time cannot be achieved. On the other hand, if a film quantity is reduced in order to achieve the rapid drug release, the initial drug release cannot be suppressed and the unpalatable taste cannot be masked. In other words, only by coating the particles containing the drug with the water-insoluble polymer, both of "the suppressing of the initial drug release" and "the subsequent rapid drug release" cannot be achieved.

As a method for achieving both of the suppressing of the initial drug release and the subsequent rapid drug release, for example, as described in International Publication No. WO02/96392 (Patent Literature 1), there is a method in which pharmaceutical composition particles are coated with a mixed film of a water-insoluble polymer and a water-soluble polymer. In the drug described in Patent Literature 1, until the water-soluble polymer in the coating film is dissolved, infiltration of water into an inside of the particles is suppressed, thereby allowing the drug release to be suppressed and leading to the expectation of masking of an unpalatable taste.

For the purpose of more ensuring the drug release in the gastrointestinal tract, for example, as described in Japanese Patent Application Laid-Open Publication No. 2007-63263 (Patent Literature 2), there is a technique in which pharmaceutical composition particles are coated with a gastrosoluble polymer or an enteric polymer as a water-insoluble polymer.

In addition, in Japanese Patent Application Laid-Open Publication No. 2008-214334 (Patent Literature 3), for the purpose of improving a degree of disintegration of film coated polymer, a method in which together with a water-insoluble polymer, a disintegrating agent and an aggregation preventing agent are used in combination is described. In Japanese Patent Application Laid-Open Publication No. 2008-260712 (Patent Literature 4), a method in which together with a water-insoluble polymer, an acidic substance or a basic substance is used in combination is described. In Japanese Patent Application Laid-Open Publication No. 2011-063627 (Patent Literature 5), a method in which together with a water-insoluble polymer, a saccharide or saccharides is used in combination is described.

Japanese Patent Application Laid-Open Publication No. 2000-191519 (Patent Literature 6) disclosed rapid release granules which are prepared by coating core granules containing a drug with a two-layered coating layer. In Patent Literature 6, it is disclosed that initial drug dissolution is suppressed by coating a mixed film of a water-insoluble substance and a water-soluble substance thereonto with a water-soluble substance as the second layer.

In addition, Japanese Patent Application Laid-Open Publication No. 2011-225468 (Patent Literature 7) described a granular pharmaceutical composition which is formulated such that a carrier is prepared as a core particle by blending a water-swellable substance used widely as a disintegrating agent; an active ingredient layer containing a drug is formed on the periphery of said carrier; and a coating layer containing a gastrosoluble polymer is formed on the periphery of said active ingredient layer. In Japanese Patent Application Laid-Open Publication No. H3-130214 (Patent Literature 8), a rapid release preparation which is formulated by preparing a core containing a drug having an unpalatable taste and a water-swellable substance and by coating the periphery of the core with a mixture of ethylcellulose and a water-soluble substance is disclosed.

### CITATION LIST

### PATENT LITERATURE

[Patent Literature 1] International Publication No. WO02/96392
[Patent Literature 2] Japanese Patent Application Laid-Open Publication No. 2007-63263
[Patent Literature 3] Japanese Patent Application Laid-Open Publication No. 2008-214334
[Patent Literature 4] Japanese Patent Application Laid-Open Publication No. 2008-260712
[Patent Literature 5] Japanese Patent Application Laid-Open Publication No. 2011-063627
[Patent Literature 6] Japanese Patent Application Laid-Open Publication No. 2000-191519
[Patent Literature 7] Japanese Patent Application Laid-Open Publication No. 2011-225468
[Patent Literature 8] Japanese Patent Application Laid-Open Publication No. H3-130214

### SUMMARY OF THE INVENTION

### TECHNICAL PROBLEM

In the drug described in Patent Literature 1, after the water-soluble polymer in the film has been eluted, fine pores are formed in the film and become water passages for allowing the drug to pass therethrough. However, in a film formulated so as to have sufficient masking of an unpalatable taste or a film having a thickness which allows sufficient masking of the unpalatable taste, water passages at such a degree as to achieve rapid drug release cannot be formed. Accordingly, when in order to solve the problem of "suppressing of the initial drug release", a coating quantity is increased and a lag time is thereby devised to extend, the rapid drug release after the lag time cannot be achieved. In addition, when in order to achieve the rapid drug release, a quantity of a water-soluble polymer in the mixed film is increased, due to difficulty in suppressing an initial drug release speed, it is required to increase the coating quantity until the lag time is generated, after all, the rapid drug release cannot be achieved. In other words, in reality, it is almost impossible to concurrently achieve controlling of the lag time and a rapid dissolution speed by coating the pharmaceutical composition particles with the mixed film of the water-insoluble substance and the water-soluble substance.

In addition, even by employing each of the methods described in Patent Literatures 2 to 5, if the lag time is made sufficient, targeted rapid drug release cannot be attained. As described above, only by conducting the coating of one layer, it is difficult to concurrently solve the two problems.

In addition, it is difficult to realize a long lag time only by the water-soluble substance as described in Patent Literature 6. It is to be noted that in Patent Literature 6, nothing as to the controlling of the lag time is described.

Each of the rapid release granular composition and the rapid release preparation described in Patent Literatures 7 and 8 has problems such as a yield of particles having uniform particle diameters is not good; a problem in that a drug dissolution quantity after 15 minutes is at a degree of 55%, which is not necessarily the rapid drug release; and a problem in that a masking time of the unpalatable taste is at a degree of 30 seconds, which is not sufficient. These problems are due to the manufacturing process. In addition, in Patent Literatures 7 and 8, nothing as to the lag time is described.

As described above, in the conventional bitter taste masking method in which inner cores prepared by the water-swellable substance, which swells merely with water, such as the disintegrating agent are utilized and in the bitter taste masking method in which a film agent using the mixture of the water-insoluble substance and the water-soluble substance is utilized, both of the lag time generation and the rapid drug release after the lapse of a desired lag time cannot be achieved.

Hence, objects of the present invention are to provide pharmaceutical composition particles for oral administration which are capable of achieving both of masking of an unpalatable taste and improvement in dissolution properties; an orally disintegrating preparation including the pharmaceutical composition particles; and a method for manufacturing the pharmaceutical composition particles. In other words, the objects of the present invention are to provide the pharmaceutical composition particles for oral administration which have a sufficiently long lag time, which do not allow a drug to be eluted in the oral cavity, are capable of performing rapid drug release after the lag time, and are further capable of controlling a length of the lag time in accordance with purposes; the orally disintegrating preparation including the pharmaceutical composition particles; and the method for manufacturing the pharmaceutical composition particles.

### SOLUTION TO PROBLEM

In order to solve these problems, the present inventors have devoted themselves to studies. As a result, the present inventors found that pharmaceutical composition particles each having a multi-layer structure, each of which includes a drug-containing core particle as a central part of a particulate composition; an intermediate layer as a coating layer constituted of a gelling swelling substance; and an outer layer including a water-insoluble component as a water infiltration quantity controlling layer, coated as an outermost layer, have a sufficient lag time which gives no feeling of an unpalatable taste in the mouth at all and are capable of realizing rapid drug release after the lag time. The present inventors further found that by changing a coating quantity and a component of each of the coating layers, a length of the lag time can be controlled to a degree of 2 to 10 minutes.

A notable feature of the present invention lies in the intermediate layer which absorbs water, gels, and swells. The heretofore known bitter taste masking fine particles using a water-swellable substance which simply absorbs water and swells like a disintegrating agent have not achieved the above-mentioned satisfactory masking of the unpalatable taste. The present inventors found out a method in which the core particle is coated with the water-soluble gelling swelling substance, as the intermediate layer, which absorbs water, gels, and swells to be denatured in a pasty state and to be bloated in its volume.

The details of dissolution of the drug from the pharmaceutical composition particles according to the present invention are as described below. Water in the oral cavity passes through the outer layer including the water-insoluble substance and infiltrates into insides of the particles little by little. The water infiltrated into the insides thereof causes the gelling swelling substance of the intermediate layer to gel and swell in the pasty state. This time required for gelling and swelling becomes the lag time. The gelling swelling substance having absorbed water becomes highly viscous gel, and a volume thereof is increased. As a result, the gelling swelling substance stretches and expands the outer layer, causes film deformation such as film thinning of the outer layer and development of cracks and breakage in the outer layer, and diffuses into the water. Although the gelling swelling substance temporarily becomes the gel, thereafter, the gelling swelling substance rapidly dissolves in the water and disperses in the water. Therefore, a surface of each of the drug-containing core particles is easily exposed and rapid release of the drug is achieved.

Based on the above-described findings, the present invention is constituted as described below. In other words, each of the pharmaceutical composition particles according to the present invention includes: a drug-containing core particle; an intermediate layer containing a water-soluble gelling swelling substance and coating an outside of the core particle; and an outer layer containing a water-insoluble substance and coating an outside of the intermediate layer.

As described later in detail, by adjusting a component and a coating quantity of the outer layer and/or a component of the intermediate layer, the lag time can be controlled.

As a result of the earnest investigation made by the present inventors, it was found that the quantity of the intermediate layer does not exert a great influence on a release speed of the drug thereinside. Thus, it was found that by increasing the coating quantity of the intermediate layer to a certain degree, a sound lag time can be set. On the other hand, it was found that since the water absorption, swelling, and bloating of the intermediate layer cause the film thinning of the outer layer and the development of the cracks and breakage therein, even when the quantity and a thickness of the outer layer are increased, the drug release speed after the lag time is not reduced.

Hence, by forming an outer layer which is capable of retaining a shape thereof in the oral cavity for, for example, one to two minutes or more, pharmaceutical composition particles do not disintegrate in the oral cavity for at least one to two minutes and as a result, the unpalatable taste can be masked for at least one to two minutes. The water infiltrates through the outer layer into the insides of the particles for a lag time of this period of one to two minutes, the pharmaceutical composition particles are moved into the stomach, and thereafter, at a degree of several minutes to 10 minutes, as described above, the film thinning of the outer layer and the cracks and breakage therein are caused due to the swelling of the intermediate layer. In this way, both of controlling of the lag time and the rapid drug release can be achieved.

As described above, it is made possible to provide the pharmaceutical composition particles which are capable of achieving both of masking of the unpalatable taste and improvement in dissolution properties, that is, the pharmaceutical composition particles for oral administration which have the sufficiently long lag time which hinders the dissolution of the drug in the oral cavity; after the lag time, are capable of rapidly releasing the drug; and further, are capable of controlling the length of the lag time in accordance with purposes.

In addition, owing to the earnest investigation made by the present inventors, it was found that by using a gelling swelling substance having a viscosity of 10 mPa·s or more in a 2% aqueous solution at a temperature of 25°C as the intermediate layer, the achievement of both of controlling of the lag time and the rapid dissolution speed is made better.

Therefore, it is preferable that in the pharmaceutical composition particles according to the present invention, the gelling swelling substance has a viscosity of 10 mPa·s or more in a 2% aqueous solution at a temperature of 25°C.

In addition, it is preferable that in the pharmaceutical composition particles according to the present invention, the outer layer contains the water-insoluble substance whose content is greater than or equal to 30% by weight with respect to a total weight of the outer layer, and it is more preferable that the outer layer contains the water-insoluble substance whose content is greater than 55% by weight with respect to the total weight of the outer layer.

In the present application, swelling power of the gelling swelling substance is defined as described below. The gelling swelling substance and water are mixed and a solution which is in a glutinous starch syrup state and attains a high viscosity of 1,900 to 2,100 mPa·s at a temperature of 30°C is obtained; and at this time, a blending quantity (part by weight) of the water with respect to 100 (part by weight) of the obtained gelling swelling substance attaining the above-mentioned viscosity is defined as the swelling power (S) of the gelling swelling substance. It is preferable that in the pharmaceutical composition particles according to the present invention, a value (S value) of the swelling power of the gelling swelling substance contained in the intermediate layer is greater than or equal to 650.

In addition, it is preferable that in the pharmaceutical composition particles according to the present invention, a coating quantity of the outer layer is greater than or equal to 5% by weight and less than or equal to 50% by weight with respect to an intermediate layer-coated particle having the core particle coated with the intermediate layer.

An orally disintegrating tablet according to the present invention includes any of the above-mentioned pharmaceutical composition particles.

It is preferable that a method for manufacturing the pharmaceutical composition particles according to the present invention includes: for example, a pulverization step of pulverizing a gelling swelling substance so as to have an average particle diameter of 15 µm or less; a suspension step of obtaining a suspension by suspending the gelling swelling substance pulverized at the pulverization step in an organic solvent; an intermediate layer formation step of forming an intermediate layer on an outside of a core particle by spraying the suspension obtained at the suspension step onto the core particle containing a drug; and an outer layer formation step of forming an outer layer by coating an outside of the intermediate layer with a water-insoluble substance.

In addition, in the method for manufacturing the pharmaceutical composition particles according to the present invention, it is preferable that the organic solvent is ethanol.

Since when the gelling swelling substance is dissolved in water, the gelling swelling substance becomes a gelatinous substance which is harder than glutinous starch syrup or becomes a liquid in the pasty state, the gelling swelling substance cannot be used in granulation and a coating operation. In addition, since the gelling swelling substance is a fibrous substance, the gelling swelling substance cannot be finely pulverized by an agitator or pulverizer ordinarily used, and the gelling swelling substance as a coating material to be used as particles which are suspended in a coating solution is hardly utilized. Although a method in which the gelling swelling substance is dissolved in water at an extremely low concentration can be considered, a long time is required for coating and the method is not realistic. In addition thereto, the gelling swelling substance has been thought to cause inconvenience in that the gelling swelling substance clings to particles and a drug and thereby hinders rapid release properties of the drug. Therefore, it has been considered to be pharmaceutically most difficult that substantially sphere-shaped fine particle cores are manufactured by using the gelling swelling substance as a main substance or the gelling swelling substance is used to coat fine particles.

The present inventors found out that the gelling swelling substance is finely pulverized so as to have the average particle diameter of 15 µm or less, the resultant is suspended in the organic solvent, for example, like the ethanol to obtain a solution, and the obtained solution is used, thereby allowing the gelling swelling substance to be used in the intermediate layer. In particular, it is considered that since the gelling swelling substance is finely pulverized so as to have the average particle diameter of 15 µm or less and is thereby coated in a homogeneous film state, after the lag time, the gelling swelling substance rapidly disperses in the water and the core particles are rapidly exposed in the water.

As described above, it is made possible to provide the method for manufacturing the pharmaceutical composition particles which are capable of both of the masking of the unpalatable taste and improvement in the dissolution properties, that is, the pharmaceutical composition particles for oral administration which have the sufficiently long lag time causing the drug not to be dissolved in the oral cavity, are capable of rapidly releasing the drug after the lag time, and are further capable of controlling the length of the lag time in accordance with purposes.

### BRIEF DESCRIPTION OF THE DRAWINGS

FIG. 1 is a graph showing results of a dissolution test as to fine particles in Example 5 in which the numbers of revolutions of a paddle are 50 rpm and 100 rpm.
FIG. 2 is a graph showing results of a dissolution test as to fine particles in Example 7 in which the numbers of revolutions of a paddle are 50 rpm and 100 rpm.
FIG. 3 is a graph showing results of a dissolution test as to fine particles with pH 1.2 and pH 6.8 in Examples 2 and 3.

### DESCRIPTION OF EMBODIMENTS

Hereinafter, an embodiment of the present invention will be described.

"Pharmaceutical composition particles" in the present invention mean a particulate composition containing a drug, which can be provided in various oral administration dosage forms.

"Suppressing of drug release" or "suppressing of initial drug dissolution" in the present invention means that in a Paddle method with 50 rpm according to the Japanese Pharmacopeia Dissolution Test in which a test fluid supposing an intraoral condition is used, a dissolution rate of a drug is suppressed to a range of 0% to 15%. Hereinafter, a period of time for which a drug dissolution rate is within the above-mentioned range is referred to as a "lag time".

When the particles according to the present invention are retained in the oral cavity for a fixed time, saliva moves through outer layers thereof into the particles and a gelling swelling substance swells. Even in particles whose gelling swelling substance is softened and dissolved little by little and whose 0% to 15% of a drug thereinside is dissolved in 900 mL of a dissolution test fluid for two minutes, the gelling swelling substance comes to be in a gelling state inside the outer layers and remains in a clayey state by a small quantity of saliva being present in the oral cavity and is not diffused into the oral cavity, and naturally, the drug inside the particles is not diffused in the oral cavity, thereby allowing a bitter taste in the oral cavity to be masked.

According to the findings made by the present inventors, in order to mask an unpalatable taste of a drug for one to two minutes upon taking the pharmaceutical composition particles, a lag time of approximately two minutes is required. Also in the case of the pharmaceutical composition particles according to the present invention, if the condition of the above-mentioned dissolution rate in the dissolution test is satisfied, masking of the unpalatable taste of the drug is achieved. It is to be noted that when upon the test with 50 rpm, the gelling swelling substance is exposed to the outer layers of the particles, due to their viscosity, a phenomenon in which the particles are mutually aggregated and deposited on a round-bottom portion of a container for the dissolution test and the drug comes to be hardly released may occur. With regard to the pharmaceutical composition particles which caused the above-mentioned phenomenon, in order to prevent the aggregation and the deposition, tests with 100 rpm were carried out.

In addition, the expression of "rapidly releasing a drug" or "rapid drug release" in the present invention means drug release conditions under which sufficient drug efficacy development can be expected. In other words, in dissolution tests using a test fluid supposing a gastrointestinal tract fluid, it is at least required that a drug dissolution rate attained after 60 minutes from the start of each of the tests is greater than or equal to 90%, and when more rapid release is demanded, it is preferable that a drug dissolution rate after 30 minutes from the start of each of the tests is greater than or equal to 80%. In each of these dissolution tests, in a case where a drug dissolution rate does not reach each of the above-mentioned rates, it means that depending on a drug, absorption of the drug in an upper gastrointestinal tract is reduced and sufficient drug efficacy development cannot be expected.

The "unpalatable taste" in the present invention means specifically a bitter taste, a rough taste, a harsh taste, an acid taste, an astringent taste, a pungent taste, and the like.

As "the test fluid supposing the intraoral condition" in the present invention, according to the finding that intraoral pH is weakly acidic, the 2nd fluid in the Japanese Pharmacopeia Dissolution Test (a phosphoric acid buffer solution with pH 6.8) was used. As "the test fluid supposing the gastrointestinal tract fluid", the 1st fluid in the Japanese Pharmacopeia Disintegration Test in consideration of a variation in intragastric pH (a hydrochloric acid buffer solution with pH 1.2), or the 2nd fluid in the Dissolution Test was used.

Hereinafter, a constitution and the like of each of the pharmaceutical composition particles according to the present invention will be described.

A "core particle containing a drug" in the present invention means a particle constituted of only a drug and a particle constituted of a drug and one kind or two or more kinds of additives, and as the core particle, a particle whose core containing no drug is coated with a drug is also included.

An "intermediate layer" in the present invention means a coating layer which is present between the core particle containing the drug and an outer layer and contains one kind or two or more kinds of gelling swelling substances. Coating of the intermediate layer can be applied directly onto the core particle containing the drug. In addition, coating of a component or components which do not hinder generation of a lag time and the subsequent rapid drug release may be applied as one layer or two or more layers of a coating layer or coating layers onto the core particle previously containing the drug and thereafter, the coating of the intermediate layer may be applied. Although the intermediate layer is formed of the gelling swelling substance as an essential constituent component, two or more kinds of gelling swelling substances may be mixedly used. In addition, it is free to apply coating of the intermediate layer by dividing the intermediate layer into two layers.

It is indispensable for the gelling swelling substance used in the present invention to be a substance which absorbs water, gels in a pasty state, and swells. As the gelling swelling substance, for example, there are carboxymethylcellulose sodium, polyethylene oxide, sodium polyacrylate, sodium alginate, propylene glycol alginate, xanthan gum, carrageenan, guar gum, tara gum, pectin, hydroxypropylmethylcellulose, hydroxypropylcellulose, hydroxyethylcellulose, methylcellulose, carboxyvinyl polymer, locust bean gum, tamarind seed gum, gum arabic, karaya gum, agar, gelatin, polyvinyl alcohol or a copolymer whose one part is the polyvinyl alcohol, and the like. One kind or two or more kinds of these gelling swelling substances are used as the intermediate layer, and coating thereof is applied.

These gelling swelling substances are dissolved in water, become paste-like, and become a solution having a high viscosity. Because of this, it is basically impossible to coat individual particles with the gelling swelling substance or substances, with water used as a solvent. Therefore, for example, the gelling swelling substance or gelling swelling substances are pulverized so as to have an average particle diameter of 15 µm or less or preferably, of 10 µm or less, and the pulverized gelling swelling substance or gelling swelling substances are suspended in an organic solvent such as ethanol, and the core particles are coated with the suspended gelling swelling substance or gelling swelling substances. At this time, it is free to blend water in the organic solvent in a range in which the suspension is not hindered. In addition, for the purpose of making adhesion of the gelling swelling substance or gelling swelling substances better, it is free to concurrently blend and use through dissolution or the like a binder such as hydroxypropylcellulose, hydroxypropylmethylcellulose, polyvinylpyrrolidone, copolyvidone, macrogol, ethylcellulose, polyvinylacetal diethylaminoacetate, hydroxypropylmethylcellulose, and hypromellose phthalate which are used widely as a binder for coating. It is to be noted that as long as the core particles can be coated with the gelling swelling substance or gelling swelling substances, a manufacturing method is not limited thereto.

In addition, for the purpose of adjusting a dissolution rate or the like of the gelling swelling substance or gelling swelling substances used for the intermediate layer, it is free to blend the intermediate layer with a saccharide or sugar alcohol such as mannitol, erythritol, and xylitol; organic acid such as citric acid, tartaric acid, and malic acid; or the like and to coat the core particles therewith.

Viscosities and swelling power of the above-mentioned gelling swelling substances extremely largely differ among product items and grades thereof. Accordingly, a blending quantity of the gelling swelling substance or gelling swelling substances in the intermediate layer and a coating quantity of the intermediate layer with which the drug-containing core particles are coated cannot be sweepingly determined. However, daringly speaking, it is preferable that a blending quantity of the gelling swelling substance or gelling swelling substances in the intermediate layer is greater than or equal to 20% and preferably, is greater than or equal to 40%. In addition, it is preferable that a coating quantity of the intermediate layer is greater than or equal to 5% by weight with respect to the drug-containing core particle and in particular, is greater than or equal to 10% by weight with respect thereto; and it is preferable that the coating quantity of the intermediate layer is less than or equal to 75% by weight and in particular, is less than or equal to 60% by weight with respect thereto. If the coating quantity is lower than 5% by weight, there is concern that no sufficiently long lag time may be generated.

The "outer layer" in the present invention is a coating layer constituted of one kind or two or more kinds of a water-insoluble substance or water-insoluble substances and is a layer which may contain one kind or two or more kinds of a water-conducting adjusting substance or water-conducting adjusting substances. Coating of the outer layer is applied further to the coating of the intermediate layer containing the gelling swelling substance or gelling swelling substances. By controlling a speed of infiltration of water into an inside of the pharmaceutical composition particle, the outer layer adjusts a water-absorbing gelling swelling speed of the intermediate layer and thereby generates a lag time.

The coating of the outer layer may be applied directly onto the intermediate layer. Coating of a component or components which do not hinder the generation of the lag time and the subsequent rapid drug release may be applied onto the intermediate layer as a coating layer of one layer or two or more layers, and thereafter, coating of the outer layer may be applied. Coating of a component or components which do not hinder the generation of the lag time and the subsequent rapid drug release may be applied onto the outer layer as a coating layer of one layer or two or more layers. In addition, since the purpose of the outer layer is to control the speed of infiltration of water, in accordance with purposes of controlling, the outer layer may be one layer or may be a plurality of layers consisting of two or more layers, causing no problem.

The water-insoluble substance used for forming the outer layer is one of essential components, which is applied onto the outer layer to control the speed of infiltration of water, and has dissolving properties, with the water-insoluble substance being thought to be hard to dissolve, extremely hard to dissolve, or practically insoluble in water. As the water-insoluble substance, specifically, cited are ethylcellulose, acetylcellulose, cellulose acetate phthalate, carboxymethylethylcellulose, hydroxypropylmethylcellulose acetate succinate, hydroxypropylmethylcellulose phthalate, dimethylaminoethyl methacrylate-methyl methacrylate copolymer, methyl acrylate-methacrylic acid copolymer, an ethyl acrylate-methyl methacrylate copolymer dispersion liquid, aminoalkyl methacrylate copolymer RS, dry methacrylic acid copolymer LD, aminoalkyl methacrylate copolymer E, methacrylic acid copolymer L, methacrylic acid copolymer LD (an aqueous dispersion liquid), methacrylic acid copolymer S, polyvinylacetal diethylaminoacetate, dry milky white lac, shellac, zein, higher fatty acid such as stearic acid, higher alcohol such as cetanol and stearyl alcohol, a low melting point substance such as carnauba wax, beeswax, and paraffin, whose melting point is 30°C to 120°C, ester of higher fatty acid and polyhydric alcohol such as sucrose fatty acid ester, fat such as hydrogenated castor oil, synthetic wax, talc, a lubricant such as magnesium stearate, and the like, but the water-insoluble substance is not limited thereto. One kind or two or more kinds of the water-insoluble substances can also be appropriately combined to be used. In addition, it is also free to mix a plasticizer such as castor oil, dibutyl phthalate, and triethyl citrate to be used.

In addition, upon forming the outer layer, the water-insoluble substance can be blended with a water-soluble substance, a hydrophilic substance, or the like as the water-conducting adjusting substance. The water-conducting adjusting substance referred to herein is a component which is blended together with the water-insoluble substance in the outer layer in order to adjust the speed of infiltration of water and a quantity of infiltration, is a component which is easily soluble in water, and is a component such as a disintegrating agent, which is hydrophilic and allows water to easily pass therethrough. Specifically, as examples of the water-conducting adjusting substance in the outer layer according to the present invention, cited are pregelatinized starch, casein sodium, a carboxyvinyl polymer, sodium carboxymethyl starch, sucrose fatty acid ester, hydroxypropylcellulose, hydroxypropylmethylcellulose, methylcellulose, hydroxyethylcellulose, pullulan, polyvinylpyrrolidone, copolyvidone, polyoxyethylene-polyoxypropylene glycol, a polyvinyl alcohol-polyethylene glycol graft copolymer, polyvinyl alcohol, macrogol, polyethylene oxide, amino acid such as glycine and alanine, a sweetening agent such as glycyrrhizic acid, a saccharide such as dextrin and lactose, sugar alcohol such as mannitol and xylitol, microcrystalline cellulose, crospovidone, triethyl citrate, and the like, but the water-conducting adjusting substance is not limited thereto. In addition, one kind or two or more kinds of the water-conducting adjusting substances can also be appropriately combined to be used.

As a composition ratio of the water-insoluble substance and the water-conducting adjusting substance in the outer layer according to the present invention, in accordance with purposes of physical properties of a drug, an absorption site, kinds of preparations, and the like, a ratio suitable for achieving the purposes is selected. It is to be noted that in a case where in the pharmaceutical composition particles according to the present invention, water permeability of the outer layer is high (a case where a blending quantity of the water-conducting substance blended together with the water-insoluble substance is large), there may be a case where the gelling swelling substance with which the intermediate layer is coated is rapidly exposed through fine pores of the outer layer to an inside of the oral cavity, and due to its viscosity, the particles solidifies inside the oral cavity and sticking thereof onto an oral mucosa occurs. Accordingly, it is preferable that a content of the water-insoluble substance in the outer layer is greater than or equal to 30% by weight, and more preferably, the content thereof is greater than or equal to 50% by weight, and in particular, it is preferable that the content thereof exceeds 55% by weight. In addition, if the ratio of the water-insoluble substance is lower than 30% by weight, there is concern that a speed at which the water infiltrates into the pharmaceutical composition particles cannot be sufficiently controlled and a sufficiently long lag time cannot be generated.

Also as a coating quantity of the outer layer in the present invention, a quantity suitable for achieving the objects of the present invention is selected. Specifically, it is preferable that the coating quantity thereof is greater than or equal to 5% by weight with respect to the intermediate layer-coated particles having the core particle coated with the intermediate layer, and in particular, it is preferable that the coating quantity thereof is greater than or equal to 7% by weight with respect thereto; and in addition, it is preferable that the coating quantity thereof is less than or equal to 50% by weight with respect thereto, and in particular, it is preferable that the coating quantity thereof is less than or equal to 30% by weight with respect thereto. If the coating quantity is lower than 5% by weight, there may be a case where coating onto a surface of the pharmaceutical composition particle is not applied uniformly, and there may be a case where due to the extremely thin outer layer, the speed at which the water infiltrates into the pharmaceutical composition particle cannot be sufficiently controlled, and there may be a case where the sufficiently long lag time cannot be generated, and a phenomenon in which the particles adheres to the inside of the oral cavity may occur. In addition, if the coating quantity is excessively large, there may be a case where the rapid drug release after the lag time is not achieved.

The drug used in the present invention is not particularly limited as long as the drug is an active component which is therapeutically or prophylactically effective. As pharmaceutically active components, for example, cited are hypnotics and sedatives; sleep inducing drug; migraine drug; anxiolytic; antiepileptic drug; antidepressant drug; antiparkinsonian drug; psychoneurotic drug; drug for the central nervous system; local anesthetic; a skeletal muscle relaxant; autonomic agents; antipyretic analgesic antiphlogistic; antispasmodics; antidizziness drug; cardiotonic drug; antiarrhythmic agents; diuretic; hypotensive drug; vasoconstrictors; vasodilators; cardiovascular agents; hypolipidemic drug; a respiratory stimulant; antitussive drug; expectorants; antitussive expectorant agents; a bronchodilator; antidiarrheal drug; an intestinal regulator; antiulcer drug; stomachics and digestives; an antacid; cathartic drug; choleretic drug; agents for digestive organs; adrenal hormone preparations; hormone drug; agents for urinary organs; vitamin preparations; hemostatic drug; drug for liver diseases; therapeutic drug for gout; antidiabetic drug; anti-histamine drug; antibiotic drug; antibacterial drug; antineoplastic drug; chemotherapeutic drug; multi-ingredient cold medication; nutritional fortification health drug; drug for osteoporosis; and the like.

A blending quantity of the drug in the pharmaceutical composition particle is not particularly limited. It is preferable that the blending quantity thereof is greater than or equal to 0.5% by weight with respect to the whole quantity of the pharmaceutical composition particle. In addition, it is preferable that the blending quantity thereof is less than or equal to 80% by weight with respect thereto, and in particular, it is preferable that the blending quantity thereof is less than or equal to 70% by weight with respect thereto, and it is further preferable that the blending quantity thereof is less than or equal to 60% by weight with respect thereto. In this regard, however, each of the blending quantities of the drug mentioned herein is merely one example, which is applicable to the present invention, and should not be restrictively construed.

As a particle diameter of each of the pharmaceutical composition particles according to the present invention, it is preferable that the longest diameter is less than or equal to 2 mm. When a shape of each of the pharmaceutical composition particles can be approximated to a sphere, it is preferable that an average particle diameter is less than or equal to 2 mm. In addition, when each of the pharmaceutical composition particles has a shape other than the sphere, it is preferable that an average longest diameter is less than or equal to 2 mm.

The pharmaceutical composition particles according to the present invention in which the unpleasant taste is masked are particularly useful as fine particles for an orally disintegrating tablet which is retained in the oral cavity for a comparatively long time. When the pharmaceutical composition particles according to the present invention are contained in the orally disintegrating tablet, in order to reduce a rough feeling in the oral cavity, it is preferable that the pharmaceutical composition particles are prepared so as to have an average particle diameter of 350 µm or less. A more preferable average particle diameter of the pharmaceutical composition particles is 50 µm to 350 µm, and a further preferable average particle diameter thereof is 70 µm to 300 µm.

It is needless to say that the pharmaceutical composition particles according to the present invention can be manufactured by using a variety of pharmaceutical additives used as commonly used additives. As the pharmaceutical additives, for example, cited are a taste masking agent, a sweetening agent, a flavoring agent, a coloring agent, a stabilizing agent, an anti-oxidizing agent, a pH-adjusting agent, a solubilizing agent, a dissolution adjuvant, a fluidizer, a buffer agent, and the like, but the pharmaceutical additives are not limited thereto.

Required quantities of the pharmaceutical composition particles according to the present invention are blended as they are, thereby allowing a variety of forms of orally administrable medicinal compositions to be manufactured. The preparations referred to herein are powder, granules, tablets, troches, dry syrup preparations, and the like. There may be a case where only by blending and mixing said fine particles for formulation as they are, the powder and the granules can be formulated. When the orally disintegrating tablets recently attracting attention are manufactured, it is required to devise a formulation method in which said fine particles for formulation are blended.

Next, a method for manufacturing the pharmaceutical composition particles according to the present invention will be described.

The pharmaceutical composition particles according to the present invention are manufactured by coating drug-containing core particles with intermediate layers and outer layers. As the drug-containing core particles, particles constituted of only a drug can be used, and particles constituted of a drug and one kind or two or more kinds of additives are produced by employing the heretofore known technology and the produced particles may be used. The particles constituted of the drug and the additive or additives may be produced, for example, by mixing a drug and an appropriate excipient or excipients (for example, microcrystalline cellulose, lactose, corn starch, or the like), by adding a solution having a binder dissolved therein as needed thereto, and by conducting granulation, particle size regulation, and drying. In addition, the particles constituted of the drug and the additive or additives may be produced by spraying a solution having a drug and a binder and the like dissolved or dispersed therein onto additive particles (for example, microcrystalline cellulose (particles), refined white sugar spherical particles, mannitol spherical particles, or the like) becoming appropriate cores by means of a fluidized bed coating apparatus or the like.

As a method of coating the drug-containing core particles with the intermediate layers, it is preferable that a method in which coating is conducted by means of a machine widely used in a coating operation of the particulate composition, such as a fluidized bed coating apparatus, a tumbling coating apparatus, and a centrifugal tumbling coating apparatus is adopted. For example, a required quantity of the solution containing a coating component may be sprayed by means of a spray gun while the drug-containing core particles are caused to be flowing in the tumbling fluidized bed coating apparatus. This solution containing the coating component is prepared by dissolving or dispersing an essential component and the like in a solvent such as water, ethanol, and methanol. However, it is also possible to appropriately mix these solvents to be used. It is to be noted that needless to say, the method of coating of these layers is not limited to a wet method.

When the pharmaceutical composition particles are used to manufacture the orally disintegrating tablets, ordinarily, it is desired that an average particle diameter of the pharmaceutical composition particles is approximately 100 to 200 µm. In order to attain the average particle diameter of approximately 100 to 200 µm after the substantially sphere-shaped core particles have been coated with the intermediate layers and the outer layers with their spherical shapes being kept, it is required that an average particle diameter of a substance which is suspended in a solution used for coating is less than or equal to one tenth of that of each of the coating particles to be coated. Accordingly, it is preferable that an average particle diameter of the gelling swelling substance included in the intermediate layers is less than or equal to 15 µm; it is more preferable that the average particle diameter thereof is less than or equal to 10 µm; and it is most preferable that the average particle diameter thereof is less than or equal to 7 µm. It is to be noted that in this description, the average particle diameters are measured by means of a laser diffraction/scattering particle size distribution measuring apparatus.

However, since the gelling swelling substance is a fibrous substance, the gelling swelling substance is hardly pulverized and cannot be easily micronized. Therefore, the present inventors have devoted themselves to studies as to a pulverization method. As a result, the present inventors found that by employing a jet mill, the gelling swelling substance can be efficiently pulverized so as to attain targeted particle diameters. In addition, the present inventors found that also by employing a wet crushing method, the gelling swelling substance can be efficiently pulverized.

As a method of coating the intermediate layer-coated particles, which have been coated with the intermediate layers, with the outer layers, it is preferable that a method in which coating is conducted by means of the machine widely used in the coating operation of the particulate composition, such as the fluidized bed coating apparatus, the tumbling coating apparatus, and the centrifugal tumbling coating apparatus is adopted. For example, a required quantity of the solution containing a coating component may be sprayed by means of the spray gun while the drug-containing core particles are caused to be flowing in a tumbling fluidized bed coating apparatus. This solution containing the coating component is prepared by dissolving or dispersing an essential component and the like in a solvent such as water, ethanol, and methanol. However, it is also possible to appropriately mix these solvents to be used, or to use only water as the solvent. It is to be noted that needless to say, the method of coating of these layers is not limited to the wet method.

As described above, it is preferable that the method for manufacturing the pharmaceutical composition particles according to the present invention includes: a pulverization step of pulverizing the gelling swelling substance so as to have the average particle diameter of 15 µm or less; a suspension step of obtaining a suspension by suspending the gelling swelling substance pulverized at the pulverization step in the organic solvent; an intermediate layer formation step of forming the intermediate layer on the outside of each of the drug-containing core particles by spraying the suspension obtained at the suspension step onto the outside of each of the drug-containing core particles; and an outer layer formation step of forming the outer layer by coating the outside of each of the intermediate layers with the water-insoluble substance.

In addition, it is preferable that the organic solvent is the ethanol.

Hereinafter, the orally disintegrating tablet containing the pharmaceutical composition particles according to the present invention will be described.

The orally disintegrating tablet referred to in the present invention means a preparation similar to a tablet which disintegrates within a fixed time, preferably within one minute, and more preferably within 45 seconds. For example, cited are orally disintegrating tablets containing pharmaceutical composition particles, disclosed in Japanese Patent Application Laid-Open Publication No. 2012-240917, Japanese Patent No. 4019374, Japanese Patent No. 3746167, and the like, respectively. As with these, the pharmaceutical composition particles according to the present invention can be used to make the orally disintegrating tablet, together with an appropriate excipient, disintegrating agent, binder, lubricant, and the like.

As specific examples of manufacturing methods, there are a variety of methods such as a method (1) in which the pharmaceutical composition particles are mixed as they are together with sugar or sugar alcohol and a selected disintegrating agent and are subjected to pressurizing and compressing, thereby manufacturing tablets; a method (2) in which the pharmaceutical composition particles are mixed with the sugar or the sugar alcohol, a selected disintegrating agent, and the like and are granulated by a binder solution to be particles, the particles are mixed with an appropriate other additive agent for tablets and are subjected to the pressurizing and compressing, thereby manufacturing tablets; and a method (3) in which the pharmaceutical composition particles are mixed with a saccharide having low moldability, the obtained mixture is sprayed to be coated, with a saccharide having high moldability used as a binder, and/or granulated, the resultant is subjected to low-pressure compression forming and thereafter, is humidified and dried, thereby manufacturing tablets. It is to be noted that in the case of the orally disintegrating tablets having the pharmaceutical composition particles blended therein, special consideration for destruction of the particles, hardness of the tablets, disintegrating properties, content uniformity, and the like is required, the method for manufacturing the orally disintegrating tablets should not be limited to the methods described herein, and there is no problem even when any method such as a molding method and a wet forming drying method is adopted upon tableting.

It is preferable that a blending quantity of the fine particles for formulation, that is, the pharmaceutical composition particles in a tablet is greater than or equal to 5% by weight and less than or equal to 85% by weight with respect to a tablet weight, it is more preferable that the blending quantity thereof is greater than or equal to 5% by weight and less than or equal to 70% by weight with respect thereto, and it is further preferable that the blending quantity thereof is greater than or equal to 10% by weight and less than or equal to 70% by weight with respect thereto. However, the blending quantity should not be limited thereto. If the blending quantity of the pharmaceutical composition particles is larger than 85% by weight, there may be a case where it is concerned that a strength and dissolution properties as a tablet, in particular, as an orally disintegrating tablet are not achieved.

The orally disintegrating tablet according to the present invention can be manufactured by blending a general additive agent which is ordinarily used for manufacturing tablets in addition to the pharmaceutical composition particles according to the present invention. As the excipient, sugar or sugar alcohol such as mannitol, erythritol, maltitol, and lactose; microcrystalline cellulose; calcium hydrogen phosphate; and the like can be used. As the binder, corn starch, polyvinylpyrrolidone, copolyvidone, hydroxypropylcellulose, polyvinyl alcohol, and the like can be used, and as long as the binder is an ordinary binder, the binder is not limited thereto. As the disintegrating agent, for example, carmellose, crospovidone, corn starch, partially pregelatinized starch, carmellose calcium, croscarmellose sodium, low substituted hydroxypropylcellulose, and the like which are ordinarily used can be used. In addition, it is also free to improve a feeling in taking medicine by blending a sweetening agent, a taste masking agent, and the like in the preparation.

The present invention is summarized as described below.
(1) Each of the pharmaceutical composition particles according to the present invention includes: the drug-containing core particle; the intermediate layer containing the water-soluble gelling swelling substance and coating the outside of the core particle; and the outer layer containing the water-insoluble substance and coating the outside of the intermediate layer.
(2) In each of the pharmaceutical composition particles according to the above-mentioned (1), it is preferable that the gelling swelling substance has a viscosity of 10 mPa·s or more in a 2% aqueous solution at a temperature of 25°C.
(3) In each of the pharmaceutical composition particles according to the above-mentioned (1) or (2), it is preferable that the outer layer contains the water-insoluble substance whose content is greater than 55% by weight with respect to a total weight of the outer layer.
(4) In each of the pharmaceutical composition particles according to any of the above-mentioned (1) to (3), it is preferable that a value of swelling power (S) of the gelling swelling substance is greater than or equal to 650.
(5) In each of the pharmaceutical composition particles according to any of the above-mentioned (1) to (4), it is preferable that a coating quantity of the outer layer is greater than or equal to 5% by weight and less than or equal to 50% by weight with respect to an intermediate layer-coated particle having the core particle coated with the intermediate layer.
(6) The orally disintegrating tablet according to the present invention includes the pharmaceutical composition particles according to any of the above-mentioned (1) to (5).
(7) The method for manufacturing the pharmaceutical composition particles according to the present invention includes: the pulverization step of pulverizing the gelling swelling substance so as to have the average particle diameter of 15 µm or less; the suspension step of obtaining the suspension by suspending the gelling swelling substance pulverized at the pulverization step in the organic solvent; the intermediate layer formation step of forming the intermediate layer on the outside of the core particle by spraying the suspension obtained at the suspension step onto the core particle containing a drug; and the outer layer formation step of forming the outer layer by coating the outside of each of the intermediate layers with the water-insoluble substance.
(8) In the manufacturing method according to the above-mentioned (7), it is preferable that the organic solvent is the ethanol.

### [Examples]

Hereinafter, the present invention will be more specifically described by Examples. However, the present invention is not limited to these Examples.

Preparation raw materials used in experiments are as follows: hydroxypropylmethylcellulose 2910 (TC-5E, viscosity: 3 mPa·s, Shin-Etsu Chemical Co., Ltd.); hydroxypropylmethylcellulose 2910 (TC-5R, viscosity: 5.8 mPa·s, Shin-Etsu Chemical Co., Ltd.); hydroxypropylmethylcellulose 2910 (TC-5S, viscosity: 15.2 mPa·s, Shin-Etsu Chemical Co., Ltd.); carmellose sodium (CELLOGEN F-5A, viscosity: 4 mPa·s, DKS Co. Ltd.); carmellose sodium (CELLOGEN F-7A, viscosity: 15 mPa·s, DKS Co. Ltd.); carmellose sodium (CELLOGEN PR-S, viscosity: 28 mPa·s, DKS Co. Ltd.); carmellose sodium (CELLOGEN F-SC, viscosity: 400 mPa·s, DKS Co. Ltd.); sodium alginate (KIMICA ALGIN IL-6, viscosity: 67 mPa·s^{*)}, KIMICA Corporation); xanthan gum (Ketorol CG, viscosity (in KCl): 600 mPa·s or more^{*)}, SANSHO Co., Ltd.); hydroxypropylcellulose (HPC-L,7.9 mPa·s, Nippon Soda Co., Ltd.); low substituted hydroxypropylcellulose (L-HPC NBD-020, Shin-Etsu Chemical Co., Ltd.); polyvinylpyrrolidone (PVP-K30, viscosity: 3 mPa·s or less, BASF Japan Ltd.); ethylcellulose (ETHOCEL 7, The Dow Chemical Company); microcrystalline cellulose (particles) (CELPHERE CP102, Asahi Kasei Chemicals Corporation); D-mannitol (Pearlitol, Roquette Japan K.K.); polyvinylacetal diethylaminoacetate (AEA, Mitsubishi-Chemical Foods Corporation); aminoalkyl methacrylate copolymer RS (EUDRAGIT RS100, HIGUCHI INC.); hydroxypropylmethylcellulose acetate succinate (AQOAT AS-MG, Shin-Etsu Chemical Co., Ltd.); castor oil (KOZAKAI PHARMACEUTICAL CO., LTD.); talc (Crown Talc Pharmacopoeia PP, Matsumura Sangyo Co., Ltd.); triethyl citrate (MORIMURA BROS., INC.); D-mannitol (160C, Roquette Japan K.K.); D-mannitol (Mannit Q, Mitsubishi Shoji Foodtech Co.,Ltd.); microcrystalline cellulose (KG-802, Asahi Kasei Chemicals Corporation); crospovidone (Polyplasdone XL-10, Ashland); magnesium stearate (Taihei Chemical Industrial Co., Ltd.); and tartaric acid (Wako Pure Chemical Industries, Ltd.). It is to be noted that each of the viscosities indicated with the mark ^{*)} is a viscosity of a 1% solution at a temperature of 25°C and each of the other viscosities is a viscosity of a 2% solution at the temperature of 25°C.

### [Experimental Example 1]

Various gelling swelling substances were used; each of the gelling swelling substances and water were mixed; a blending quantity (part by weight) of the water with respect to 100 (part by weight) of each of the gelling swelling substances, which allowed a solution having a viscosity at a temperature of 30°C in a range of 1,900 to 2,100 mPa·s to be prepared, was obtained; and the blending quantity was defined to be swelling power (S) of each of the gelling swelling substances. The obtained S values were as follows: TC-5E: 317; TC-5R: 514; TC-5S: 931; CELLOGEN F-5A: 525; CELLOGEN F-7A: 953; CELLOGEN PR-S: 1,011; CELLOGEN F-SC: 2,074; KIMICA ALGIN IL-6: 2,226; Ketorol CG: 5,456; HPC-L: 590; and PVP-K30: 110.

It is to be noted that in the case of CELLOGEN F-7A whose S value was 953, because a volume of a solution in which 5 g of CELLOGEN F-7A and 45 mL of ethanol were mixed and suspended was 49.3 mL, a volume of 5 g of CELLOGEN F-7A was equivalent to 4.3 mL. Accordingly, a volume of an aqueous solution prepared by adding 953 g of water, which was equivalent to the S value, to 100 g of CELLOGEN F-7A (a volume was equivalent to 86 mL) was 1,039 mL; a viscosity thereof was 1,900 to 2,100 mPa·s; and a water absorption swelling rate became 12.1 times. In addition, in the case of the same experiment as to HPC-L, because a volume of 5 g of HPC-L was equivalent to 4.2 mL, a volume of an HPC-L aqueous solution prepared by adding 590 g of water, which was equivalent to the S value, to 100 g of HPC-L (a volume was equivalent to 84 mL), was 674 mL; a viscosity thereof was 1,900 to 2,100 mPa·s; and a water absorption swelling rate became 8.0 times. It is to be noted that similarly, a water absorption swelling rate of each of the other gelling swelling substances was calculated as follows: TC-5E: 4.7 times; TC-5R: 7.4 times; CELLOGEN PR-S: 12.5 times; and CELLOGEN F-SC: 25.7 times.

### [Test Example 1]

With regard to pharmaceutical composition particles (Examples 1 to 5 and Comparative Examples 4 and 5) using various gelling swelling substances in intermediate layers; pharmaceutical composition particles (Comparative Example 1) having no intermediate layers; pharmaceutical composition particles (Comparative Examples 2 and 3) having no outer layers; and pharmaceutical composition particles (Comparative Examples 6 and 7) using other substances in intermediate layers, instead of the gelling swelling substances, a dissolution test and evaluation of masking properties of an unpalatable taste were conducted.

### [Examples 1 to 5]

A layering solution was prepared by suspending 85 g of ambroxol hydrochloride (with an average particle diameter of approximately 3 µm) in a solution in which 20.74 g of PVP-K30 was dissolved in 826.2 g of purified water. An intermediate layer solution was prepared by suspending or dissolving 43.20 g of a finely pulverized material of each of the gelling swelling substances and the like shown in each column in Table 1 in a solution prepared by dissolving 10.80 g of HPC-L in a mixture solution of 923.48 g of ethanol and 102.61 g of purified water. A viscosity of each of the gelling swelling substances in Table 1 is a viscosity of a 2% solution at a temperature of 25°C and a viscosity indicated with the mark ^{*)} is a viscosity of a 1% solution at the temperature of 25°C.

Each of the gelling swelling substance was pulverized by means of a jet mill so as to have an average particle diameter of 7 µm or less. Average particle diameters of the pulverized gelling swelling substances were as follows: CELLOGEN F-7A: 4.0 µm; CELLOGEN PR-S: 4.7 µm; CELLOGEN F-SC: 6.5 µm; KIMICA ALGIN IL-6: 3.8 µm; and Ketorol CG: 3.1 µm. It is to be noted that with regard to the intermediate layer solution of NBD-020, for the purpose of preventing clogging of a spray nozzle, a mixture solution of 1,571.4 g of ethanol and 174.6 g of purified water was used. An outer layer solution was prepared by dissolving 32.4 g of ETHOCEL 7 and 3.6 g of TC-5E in a mixture solution of 291.63 g of ethanol and 32.4 g of purified water.

**[Table 1]**

| | Gelling swelling substance, etc. in intermediate layer | Viscosity of gelling swelling substance (mPa·s) | Water-insoluble substance in outer layer |
|---|---|---|---|
| Example 1 | CELLOGEN F-7A | 15 | ETHOCEL 7 |
| Example 2 | TC-5S | 15.2 | ETHOCEL 7 |
| Example 3 | CELLOGEN PR-S | 28 | ETHOCEL 7 |
| Example 4 | CELLOGEN F-SC | 400 | ETHOCEL 7 |
| Example 5 | KIMICA ALGIN IL-6 | 67^{*)} | ETHOCEL 7 |
| Comparative Example 1 | - | - | ETHOCEL 7 |
| Comparative Example 2 | TC-5S | 15.2 | - |
| Comparative Example 3 | CELLOGEN PR-S | 28 | - |
| Comparative Example 4 | TC-5E | 3 | ETHOCEL 7 |
| Comparative Example 5 | HPC-L | 7.9 | ETHOCEL 7 |
| Comparative Example 6 | D-mannitol | - | ETHOCEL 7 |
| Comparative Example 7 | L-HPC NBD-020 | - | ETHOCEL 7 |

Inputted into a tumbling fluidized bed coating granulating machine (manufactured by Powrex Corporation: MP-01 model) was 0.85 kg of CELPHERE CP-102; 931.94 kg of the layering solution was sprayed thereto for coating, with agitating and fluidizing being conducted; drying was conducted; and thereafter, sifting was conducted by means of a 42-mesh sieve and a 150-mesh sieve, thereby obtaining drug layering particles (core particles). The drug layering particles were manufactured by repeating the experiment under the same conditions several times to be used in the below-described experiments.

Next, 180 g of the drug layering particles were inputted into the tumbling fluidized bed coating granulating machine (MP-01 model); 1,080.1 g of the intermediate layer solution was sprayed thereto for coating, with agitating and fluidizing being conducted; and drying was conducted, thereby obtaining intermediate layer-coated particles. Subsequently, 180 g of the intermediate layer-coated particles were inputted into the tumbling fluidized bed coating granulating machine (MP-01 model); 360 g of the outer layer solution was sprayed thereto for coating, with agitating and fluidizing being conducted; and drying was conducted, thereby obtaining outer layer-coated particles (pharmaceutical composition particles) in Examples 1 to 5.

### [Comparative Examples 1 to 7]

Particles in Comparative Example 1 were prepared by directly coating the outer layer solution onto the drug layering particles without coating the gelling swelling substance layer (having no intermediate layers). The intermediate layer-coated particles prepared in Example 2 were used as particles in Comparative Example 2 (with no coating of the outer layers). The intermediate layer-coated particles prepared in Example 3 were used as particles in Comparative Example 3 (with no coating of the outer layers). By using TC-5E, HPC-L, D-mannitol, and L-HPC (NBD-020) respectively, instead of the intermediate layer gelling swelling substances used in Examples 1 to 5, particles were prepared by employing the same method as in Examples 1 to 5, thereby obtaining outer layer-coated particles (pharmaceutical composition particles) in Comparative Example 4, Comparative Example 5, Comparative Example 6, and Comparative Example 7.

With regard to the particles obtained in Examples 1 to 5 and Comparative Examples 1 to 7, a dissolution test and evaluation of masking properties of an unpalatable taste were conducted as described below.

### [Dissolution Test]

A dissolution test was conducted by taking outer layer-coated particles including 20 mg of ambroxol hydrochloride, by means of an automatic 6-channel dissolution test apparatus (manufactured by TOYAMA SANGYO CO., LTD.), and in accordance with Japanese Pharmacopoeia Method 2. As a test fluid, 900 mL of the 2nd fluid in the Japanese Pharmacopeia Dissolution Test was used. It is to be noted that the number of revolutions of a paddle was 50 rpm or 100 rpm. Results of the dissolution test are shown in Table 2.

**[Table 2]**

| | Number of revolutions of paddle (rpm) | Dissolution rate (%) | | | | |
|---|---|---|---|---|---|---|
| | | 2 minutes | 15 minutes | 30 minutes | 45 minutes | 60 minutes |
| Example 1 | 50 | 7.5 | 66.1 | 82.9 | 88.2 | 91.5 |
| Example 2 | 50 | 6.7 | 56.0 | 83.6 | 87.5 | 92.0 |
| Example 3 | 50 | 6.0 | 70.6 | 85.5 | 90.5 | 92.0 |
| Example 4 | 100 | 5.1 | 82.6 | 92.5 | 95.8 | 97.0 |
| Example 5 | 100 | 2.7 | 57.5 | 82.5 | 89.0 | 92.9 |
| Comparative Example 1 | 50 | 3.6 | 28.1 | 48.2 | 55.8 | 62.7 |
| Comparative Example 2 | 50 | 58.1 | 98.5 | 98.7 | - | - |
| Comparative Example 3 | 50 | 53.6 | 100.3 | 100.3 | - | - |
| Comparative Example 4 | 50 | 1.1 | 38.9 | 62.6 | 74.4 | 81.2 |
| Comparative Example 5 | 50 | 2.1 | 22.9 | 42.9 | 58.7 | 69.0 |
| Comparative Example 6 | 50 | 0.9 | 21.2 | 43.4 | 57.9 | 68.5 |
| Comparative Example 7 | 50 | 2.6 | 26.1 | 50.6 | 67.7 | 78.7 |

### [Evaluation of masking properties of unpalatable taste]

Tastes of the outer layer-coated particles including the ambroxol hydrochloride and of ambroxol hydrochloride bulk powder were evaluated by employing the below-described evaluation method based on the below-described evaluation criteria. Results are shown in Table 3.

### <Evaluation method>

Four healthy adult men (panelist 1 to panelist 4) took and kept film-coated particles containing ambroxol hydrochloride whose quantity was equivalent to 15 mg, in their mouths for two minutes; thereafter, spat out these; and evaluated an unpalatable taste thereof after one minute and after two minutes. In addition, the four healthy adult men took and kept ambroxol hydrochloride bulk powder in their mouths for 10 seconds; thereafter, spat out this; and evaluated an unpalatable taste thereof.

### <Evaluation criteria of unpalatable taste>

-: He felt no unpalatable taste.
±: He slightly felt the taste but it was allowable.
+: He felt the unpalatable taste.
++: He strongly felt the unpalatable taste.

**[Table 3]**

| | | Example | | | | | Comparative Example | | | | | | |
|---|---|---|---|---|---|---|---|---|---|---|---|---|---|
| | | 1 | 2 | 3 | 4 | 5 | 1 | 2 | 3 | 4 | 5 | 6 | 7 |
| 1 minute | Panelist 1 | - | - | - | - | - | ± | ++ | ++ | ± | - | - | - |
| | Panelist 2 | - | - | - | - | - | - | ++ | ++ | - | - | - | - |
| | Panelist 3 | - | - | - | - | - | ± | ++ | ++ | - | - | - | - |
| | Panelist 4 | - | - | - | - | - | - | ++ | ++ | - | - | - | - |
| 2 minutes | Panelist 1 | - | - | ± | ± | - | + | | | ± | - | - | ± |
| | Panelist 2 | - | - | ± | - | - | ± | | | - | - | - | - |
| | Panelist 3 | - | - | - | - | - | + | | | - | - | - | - |
| | Panelist 4 | - | - | - | - | - | ± | | | - | - | - | - |

With regard to the ambroxol hydrochloride bulk powder, all of the panelists felt the taste as indicated by ++ from after one minute.

With regard to all of samples in Examples 1 to 5, even after two minutes after all of the samples were taken and kept in their mouths, as to a bitter taste in their oral cavities, the unpalatable taste causing any problems was not felt and a clear effect of suppressing the unpalatable taste was obtained. In addition, also in the dissolution test, all of the samples exhibited the dissolution rates of 80% or more after 30 minutes and the dissolution rates of 90% or more after 60 minutes and had drug release properties from which rapid and sufficient drug efficacy development can be expected.

In contrast to this, with regard to the pharmaceutical composition particles in Comparative Example 1 which were obtained by directly coating the core particles with the outer layers without coating the intermediate layers, the plurality of panelists felt the unpalatable taste even after one minute; in the dissolution test, the drug release did not reach 90% even after 60 minutes; and it was found that the sufficient drug efficacy development cannot be expected. In addition, with regard to the particles in Comparative Examples 2 and 3, the results of the dissolution test showed that each of the preparations exhibited the drug release of 50% or more after two minutes; when the preparations were kept in their oral cavities, the strong unpalatable taste was observed within 30 seconds; and the unpalatable taste was not masked at all.

With regard to the pharmaceutical composition particles in Comparative Examples 4 and 5 in which as the gelling swelling substances, TC-5E and HPC-L having weak water-absorbing swelling power were used, although the unpalatable taste was masked, because the gelling swelling power was weak, capability of denaturing the outer layers was weak; in the dissolution test, the drug release did not reach 90% even after 60 minutes; and it was found that the sufficient drug efficacy development cannot be expected.

In addition, with regard to the pharmaceutical composition particles in Comparative Example 6 in which instead of the gelling swelling substance, D-mannitol which was the sugar alcohol was used and also the pharmaceutical composition particles in Comparative Example 7 in which instead of the gelling swelling substance, L-HPC (NBD-020) as a water-insoluble swelling substance was used, although the unpalatable taste was masked, because of weak capability of denaturing the outer layers, of week diffusing power due to the water-insolubility, and of others, in the dissolution test, the drug release did not reach 90% even after 60 minutes and it was found that the sufficient drug efficacy development cannot be expected.

### [Test Example 2]

In the pharmaceutical composition particles according to the present invention, when cracks and the like are caused in outer layers upon conducting a dissolution test, a gelling swelling substance is exposed to surfaces thereof and mutual aggregation of the particles occurs due to viscous properties thereof inside a dissolution test fluid. Therefore, when the number of paddle revolutions upon conducting the dissolution test is low (50 rpm), wet aggregates having viscous properties are formed in the test fluid, and true dissolution data cannot be obtained. Thus, for the purpose of loosening the aggregation and obtaining the true dissolution data, comparative experiments with the number of paddle revolutions of 100 rpm were conducted.

### [Example 6]

Particles in Example 6 were prepared by sampling the particles in Example 4 at the point of time when the particles were coated with three fourths of the quantity of the outer layer solution and drying the sampled particles.

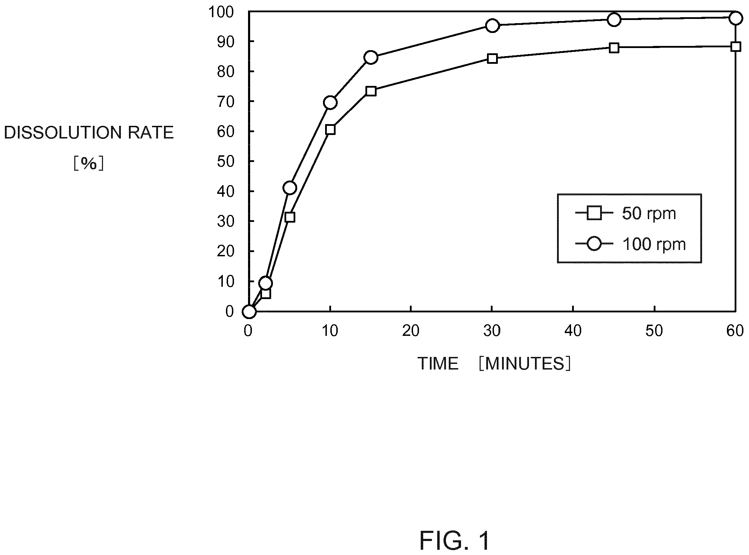

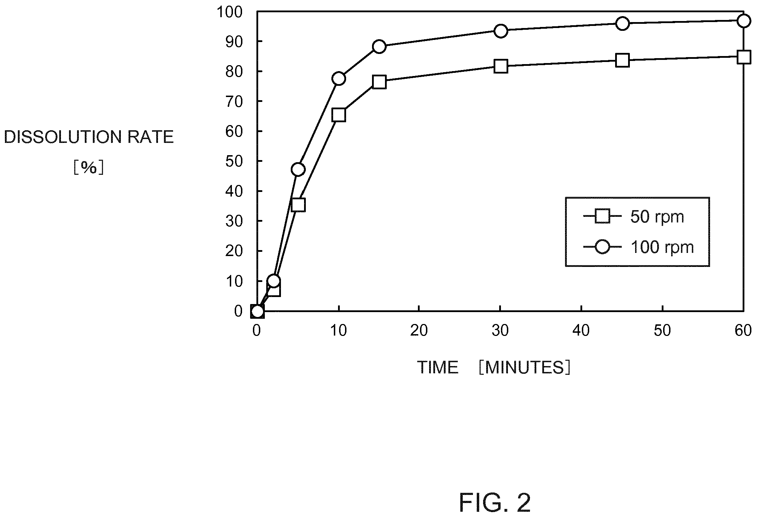

The dissolution tests were conducted by using the particles in Example 4 and the particles in Example 6, by means of an automatic 6-channel dissolution test apparatus (manufactured by TOYAMA SANGYO CO., LTD.), and in accordance with the Japanese Pharmacopoeia Method 2. As a test fluid, 900 mL of the 2nd fluid in the Japanese Pharmacopeia Dissolution Test was used. The numbers of revolutions of a paddle were 50 rpm and 100 rpm. Results of the dissolution tests as to the particles in Example 4 and the particles in Example 6 are shown in FIG. 1 and FIG. 2, respectively. It is to be noted that all of the panelists which took and kept the particles in Example 6 in their oral cavities for two minutes felt no bitter taste.

As shown in FIG. 1 and FIG. 2, with regard to both of the particles in Example 4 and the particles in Example 6, there are no large differences in the dissolution up to two minutes between 50 rpm and 100 rpm. On the other hand, with 50 rpm, dissolution speeds at and after 10 minutes were slowed; even at 60 minutes, the particles in Example 4 and the particles in Example 6 were not completely dissolved; and at approximately 10 minutes, the aggregation of the particles in both thereof occurred. In addition, with 100 rpm, 85% or more thereof was dissolved at 15 minutes, aggregation properties were loosened, and true dissolution was reflected.

Accordingly, when with the number of paddle revolutions of 50 rpm, strong mutual aggregation of the particles is recognized, for the purpose of loosening the aggregation, tests with 100 rpm are of significance.

In addition, from these results of the tests, it was found that the pharmaceutical composition particles according to the present invention can be provided as particles which hardly give the feeling of a bitter taste in the oral cavity for two minutes and have dissolution properties exhibiting 85% or more dissolution after 15 minutes in the dissolution tests.

### [Test Example 3]

With regard to the pharmaceutical composition particles according to the present invention, drug release properties of solutions having different pH values were examined.

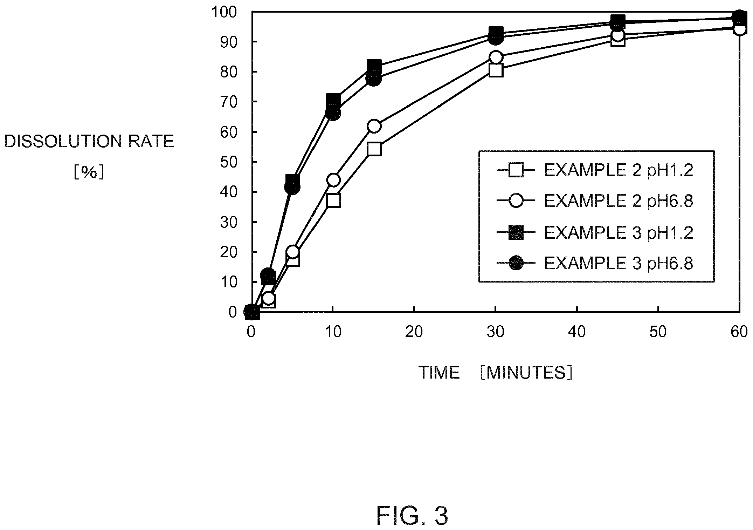

With regard to the particles in Example 2 and the particles in Example 3, dissolution tests were conducted by means of an automatic 6-channel dissolution test apparatus (manufactured by TOYAMA SANGYO CO., LTD.) in accordance with the Japanese Pharmacopoeia Method 2. As test fluids, 900 mL of the 2nd fluid in the Japanese Pharmacopeia Dissolution Test (with pH 6.8) and 900 mL of the 1st fluid in the Japanese Pharmacopeia Disintegration Test Method (with pH 1.2) were used. As the number of revolutions of a paddle, in order to prevent the aggregation of the pharmaceutical composition particles in the dissolution test fluids, 100 rpm was adopted. Results of the dissolution tests of the particles in Example 2 and the particles in Example 3 are shown in Table 4 and FIG. 3.

**[Table 4]**

| | pH | Dissolution rate (%) | | | | | | |
|---|---|---|---|---|---|---|---|---|
| | | 2 minutes | 5 minutes | 10 minutes | 15 minutes | 30 minutes | 45 minutes | 60 minutes |
| Example 2 | 1.2 | 3.7 | 17.7 | 37.2 | 54.5 | 80.7 | 90.9 | 95.1 |
| | 6.8 | 4.7 | 20.2 | 44.1 | 62.1 | 85.0 | 92.5 | 94.5 |
| Example 3 | 1.2 | 11.5 | 43.7 | 70.5 | 81.9 | 92.9 | 96.9 | 97.9 |
| | 6.8 | 12.1 | 41.6 | 66.4 | 77.8 | 91.5 | 96.0 | 98.1 |

There were no differences in dissolution properties between the two test fluids, and it is seen that the pharmaceutical composition particles according to the present invention are excellent preparations which have similar drug release properties in either of the stomach and the intestinal tract.

### [Test Example 4]

The drug included in the core particles was changed (in Examples 7 to 10) and the same dissolution test and evaluation of masking properties of an unpalatable taste as in Test Example 1 were implemented.

### [Example 7]

A layering solution was prepared by suspending 30 g of sertraline hydrochloride in a solution in which 7.32 g of PVP-K30 was dissolved in a mixture solution of 66 g of purified water and 600 g of ethanol. An intermediate layer solution was prepared by suspending 75 g of CELLOGEN PR-S in a solution in which 18.75 g of HPC-L was dissolved in a mixture solution of 53.12 g of purified water and 478.13 g of ethanol. An outer layer solution was prepared by dissolving 45 g of ETHOCEL 7 and 5 g of TC-5E in a mixture solution of 45 g of purified water and 405 g of ethanol.

Inputted into a tumbling fluidized bed coating granulating machine (manufactured by Powrex Corporation: MP-01 model) was 300 g of CELPHERE CP-102; 703.32 g of the layering solution was sprayed thereto for coating, with agitating and fluidizing being conducted; drying was conducted; and thereafter, sifting was conducted by means of a 42-mesh sieve and a 150-mesh sieve, thereby obtaining drug layering particles.

Next, 250 g of the drug layering particles were inputted into the tumbling fluidized bed coating granulating machine (MP-01 model); 625 g of the intermediate layer solution was sprayed thereto for coating, with agitating and fluidizing being conducted; and drying was conducted, thereby obtaining intermediate layer-coated particles. Subsequently, 250 g of the intermediate layer-coated particles were inputted into the tumbling fluidized bed coating granulating machine (MP-01 model); 500 g of the outer layer solution was sprayed thereto for coating, with agitating and fluidizing being conducted; and drying was conducted, thereby obtaining outer layer-coated particles (pharmaceutical composition particles).

A dissolution test was conducted by taking the outer layer-coated particles including 50 mg of sertraline hydrochloride, by means of an automatic 6-channel dissolution test apparatus (manufactured by TOYAMA SANGYO CO., LTD.), and in accordance with Japanese Pharmacopoeia Method 2. As a test fluid, 900 mL of a phosphoric acid buffer solution with pH 6.8 (the 2nd fluid in the Japanese Pharmacopeia Disintegration Test Method) was used. It is to be noted that the number of revolutions of a paddle was 50 rpm. Results of the dissolution test are shown in Table 5.

### [Example 8]

A layering solution was prepared by suspending 30 g of fexofenadine hydrochloride in a solution in which 7.32 g of PVP-K30 was dissolved in a mixture solution of 50 g of purified water and 450 g of ethanol. An intermediate layer solution was prepared by suspending 75 g of CELLOGEN PR-S in a solution in which 18.75 g of HPC-L was dissolved in a mixture solution of 53.12 g of purified water and 478.13 g of ethanol. An outer layer solution was prepared by dissolving 45 g of ETHOCEL 7 and 5 g of TC-5E in a mixture solution of 45 g of purified water and 405 g of ethanol.

Inputted into a tumbling fluidized bed coating granulating machine (manufactured by Powrex Corporation: MP-01 model) was 300 g of CELPHERE CP-102; 537.32 g of the layering solution was sprayed thereto for coating, with agitating and fluidizing being conducted; drying was conducted; and thereafter, sifting was conducted by means of a 42-mesh sieve and a 150-mesh sieve, thereby obtaining drug layering particles.

Next, 250 g of the drug layering particles were inputted into the tumbling fluidized bed coating granulating machine (MP-01 model); 625 g of the intermediate layer solution was sprayed thereto for coating, with agitating and fluidizing being conducted; and drying was conducted, thereby obtaining intermediate layer-coated particles. Subsequently, 250 g of the intermediate layer-coated particles were inputted into the tumbling fluidized bed coating granulating machine (MP-01 model); 500 g of the outer layer solution was sprayed thereto for coating, with agitating and fluidizing being conducted; and drying was conducted, thereby obtaining outer layer-coated particles (pharmaceutical composition particles).

A dissolution test was conducted by taking the outer layer-coated particles including 60 mg of fexofenadine hydrochloride, by means of an automatic 6-channel dissolution test apparatus (manufactured by TOYAMA SANGYO CO., LTD.), and in accordance with Japanese Pharmacopoeia Method 2. As a test fluid, 900 mL of a phosphoric acid buffer solution with pH 6.8 (the 2nd fluid in the Japanese Pharmacopeia Disintegration Test Method) was used. It is to be noted that because mutual adhesion among the particles was observed, the number of revolutions of a paddle was set to 100 rpm. Results of the dissolution test are shown in Table 5.

### [Example 9]

A drug coating solution was prepared by suspending 20 g of talc in a solution in which 10 g of solifenacin succinate, 25 g of TC-5E, and 7 g of tartaric acid were dissolved in a mixture solution of 375 g of purified water and 375 g of ethanol. An intermediate layer solution was prepared by suspending 60 g of micronized powder of carmellose sodium (CELLOGEN PR-S) in a solution in which 20 g of HPC-L was dissolved in a mixture solution of 70 g of purified water and 630 g of ethanol. An outer layer solution was prepared by dissolving 39.6 g of ETHOCEL 7 and 4.4 g of TC-5E in a mixture solution of 176 g of purified water and 704 g of ethanol.

Inputted into a tumbling fluidized bed coating granulating machine (MP-01 model) was 200 g of microcrystalline cellulose (CELPHERE CP102); 812 g of the drug coating solution was sprayed thereto for coating, with agitating and fluidizing being conducted; and drying was conducted, thereby preparing drug-coated particles. Inputted into the tumbling fluidized bed coating granulating machine (MP-01 model) were 200 g of the drug-coated particles; 780 g of the intermediate layer solution was sprayed thereto for coating, with agitating and fluidizing being conducted; and drying was conducted, thereby preparing intermediate layer-coated particles.

Next, 220 g of the intermediate layer-coated particles were inputted into the tumbling fluidized bed coating granulating machine (MP-01 model); 924 g of the outer layer solution was sprayed thereto for coating, with agitating and fluidizing being conducted; and drying was conducted, thereby obtaining outer layer-coated particles.

A dissolution test was conducted by taking the outer layer-coated fine particles including 5 mg of the solifenacin succinate, by means of an automatic 6-channel dissolution test apparatus (manufactured by TOYAMA SANGYO CO., LTD.), and in accordance with Japanese Pharmacopoeia Method 2. As a test fluid, 900 mL of the 2nd fluid in the Japanese Pharmacopeia Dissolution Test was used. It is to be noted that the number of revolutions of a paddle was 50 rpm. Results of the dissolution test are shown in Table 5.

### [Example 10]

A drug coating solution was prepared by suspending 160 g of talc in a solution in which 80 g of solifenacin succinate, 200 g of TC-5E, and 56 g of tartaric acid were dissolved in a mixture solution of 3,000 g of purified water and 3,000 g of ethanol. An intermediate layer solution was prepared by suspending 60 g of micronized powder of carmellose sodium (CELLOGEN F-SC) in a solution in which 20 g of HPC-L was dissolved in a mixture solution of 70 g of purified water and 630 g of ethanol. An outer layer solution was prepared by dissolving 39.6 g of ETHOCEL 7 and 4.4 g of TC-5E in a mixture solution of 176 g of purified water and 704 g of ethanol.

Inputted into a tumbling fluidized bed coating granulating machine (MP-01 model) were 200 g of microcrystalline cellulose (CELPHERE CP102); 6,496 g of the drug coating solution was sprayed thereto for coating, with agitating and fluidizing being conducted; and drying was conducted, thereby preparing drug-coated particles. Inputted into the tumbling fluidized bed coating granulating machine (MP-01 model) were 200 g of the drug-coated particles; 780 g of the intermediate layer solution was sprayed thereto for coating, with agitating and fluidizing being conducted; and drying was conducted, thereby preparing intermediate layer-coated particles.

Next, 220 g of the intermediate layer-coated particles were inputted into the tumbling fluidized bed coating granulating machine (MP-01 model); 924 g of the outer layer solution was sprayed thereto for coating, with agitating and fluidizing being conducted; and drying was conducted, thereby obtaining outer layer-coated particles.

By employing the same method as in Example 9, a dissolution test was conducted. However, because mutual adhesion among the particles was observed, the number of revolutions of a paddle was set to 100 rpm. Results of the dissolution test are shown in Table 5.

**[Table 5]**

| | Number of paddle revolutions (rpm) | Dissolution rate (%) | | | | |
|---|---|---|---|---|---|---|
| | | 2 minutes | 15 minutes | 30 minutes | 45 minutes | 60 minutes |
| Example 7 | 50 | 7.6 | 68.7 | 83.5 | 90.0 | 92.9 |
| Example 8 | 100 | 5.1 | 56.3 | 84.0 | 94.1 | 96.5 |
| Example 9 | 50 | 5.5 | 72.0 | 86.5 | 91.5 | 93.2 |
| Example 10 | 100 | 4.1 | 67.4 | 83.2 | 91.4 | 96.6 |

### [Evaluation of masking properties of unpalatable taste]

Tastes of Examples 7 to 10 were evaluated as in Test Example 1. In other words, four healthy adult men (panelist 1 to panelist 4) took and kept the film-coated particles containing the drugs whose each quantity was equivalent to 15 mg, in their mouths for two minutes; thereafter, spat out these; and evaluated an unpalatable taste thereof after one minute and after two minutes. In addition, the four healthy adult men took and kept bulk powder of each of the drugs in their mouths for 10 seconds; thereafter, spat out this; and evaluated an unpalatable taste thereof.

**[Table 6]**

| | | Example 7 | Example 8 | Example 9 | Example 10 |
|---|---|---|---|---|---|
| 1 minute | Panelist 1 | - | - | - | - |
| | Panelist 2 | - | - | - | - |
| | Panelist 3 | - | - | - | - |
| | Panelist 4 | - | - | - | - |
| 2 minutes | Panelist 1 | - | ± | - | - |
| | Panelist 2 | - | ± | - | - |
| | Panelist 3 | - | - | - | ± |
| | Panelist 4 | - | - | - | ± |

With regard to the sertraline hydrochloride, the fexofenadine hydrochloride, or the solifenacin succinate, all of the panelists felt the taste as indicated by ++ from after one minute.

With regard to all of the samples in Examples 7 to 10, even after two minutes after all of the samples were taken and kept therein, as to a bitter taste in their oral cavities, the unpalatable taste causing any problems was not felt and a clear effect of suppressing the unpalatable taste was obtained. In addition, also in the dissolution test, all of the samples exhibited the dissolution rates of 80% or more after 30 minutes and the dissolution rates of 90% or more after 60 minutes and had drug release properties from which rapid and sufficient drug efficacy development can be expected.

### [Test Example 5]

A quantity of a water-insoluble polymer included in the outer layers was changed (Examples 11 to 13) and the same dissolution test and evaluation of masking properties of an unpalatable taste as in Test Example 1 were implemented.

### [Examples 11 to 13]

An outer layer solution was prepared by dissolving 33 g of ETHOCEL 7 and 27 g of TC-5E in a mixture solution of 152 g of purified water and 912 g of ethanol. Inputted into a tumbling fluidized bed coating granulating machine (MP-01 model) were 200 g of intermediate layer-coated particles prepared by employing the same method as in Example 4; 1124 g of the outer layer solution was sprayed thereto for coating, with agitating and fluidizing being conducted; and drying was conducted, thereby obtaining outer layer-coated particles. Half way therethrough, a quantity of two thirds thereof were sampled and dried, thereby obtaining outer layer-coated particles in Example 11. Outer layer-coated particles (pharmaceutical composition particles) in Example 12 were prepared by coating the whole quantity (equivalent to 30 part by weight) of the intermediate layer-coated particles with respect to the total weight of the intermediate layer-coated particles. In addition, outer layer-coated particles (pharmaceutical composition particles) in Example 13 were prepared with blending quantities of 42 g of ETHOCEL 7 and 18 g of TC-5E by coating the intermediate layer-coated particles whose content was equivalent to 20 part by weight with respect to the total weight thereof by employing the same method.

Dissolution tests were conducted by using the outer layer-coated particles in Examples 11 to 12 which were prepared such that each of the contents of the water-insoluble polymer included in the outer layers was 55% by weight with respect to the total weight of the outer layers and the outer layer-coated particles in Example 13 which were prepared such that the content of the water-insoluble polymer included in the outer layers was 70% by weight with respect thereto and by employing the same method as in Test Example 1. Results are shown in Table 7.

**[Table 7]**

| | Number of paddle revolutions (rpm) | Dissolution rate (%) | | | | |
|---|---|---|---|---|---|---|
| | | 2 minutes | 15 minutes | 30 minutes | 45 minutes | 60 minutes |
| Example 11 | 50 | 14.8 | 91.2 | 94.1 | 95.0 | 96.6 |
| Example 12 | 50 | 13.5 | 88.6 | 92.2 | 93.4 | 95.2 |
| Example 13 | 50 | 3.0 | 79.8 | 90.2 | 93.4 | 95.3 |

As the results of the dissolution tests, it was found that the tested particles were rapid-release particles whose dissolution rates were 80% or more at 15 minutes. On the other hand, with regard to the outer layer-coated particles in all of the above-shown Examples, an unpalatable taste was not felt at two minutes. However, with regard to the outer layer-coated particles in Examples 11 and 12 which were prepared such that each of the contents of the water-insoluble polymer included in the outer layers was 55% by weight with respect to the total weight of the outer layers, due to the gelling swelling substance oozing out in the oral cavity, fine particles occasionally stuck to an inside of the oral cavity. Accordingly, it was found that in particular, it is preferable that the content of the water-insoluble substance in the outer layers exceeds 55% by weight.

### [Test Example 6]

A kind of the water-insoluble polymer included in the outer layers was changed (Examples 14 to 16) and the same dissolution test and evaluation of masking properties of an unpalatable taste as in Test Example 1 were implemented.

### [Examples 14 to 16]

An EUDRAGIT RS outer layer solution was prepared by dissolving or suspending 12.5 g of EUDRAGIT RS100, 1.25 g of triethyl citrate, and 6.25 g of talc in a mixture solution of 14.7 g of purified water and 132 g of ethanol. An AEA outer layer solution was prepared by dissolving 36 g of AEA and 4 g of castor oil in 760 g of ethanol. An AQOAT outer layer solution was prepared by dissolving 40 g of AQOAT (AS-MG) in a mixture solution of 152 g of purified water and 608 g of ethanol.

Inputted into a tumbling fluidized bed coating granulating machine (MP-01 model) were 200 g of intermediate layer-coated particles prepared by employing the same method as in Example 5; each of the outer layer solutions was sprayed thereto for coating, with agitating and fluidizing being conducted; and drying was conducted, thereby obtaining outer layer-coated particles. Outer layer-coated particles in Example 14 were prepared by using 166.7 g of the EUDRAGIT RS outer layer solution with respect to 200 g of the intermediate layer-coated particles and conducting spray-coating thereof and drying. Outer layer-coated particles in Example 15 were prepared by using 800 g of the AEA outer layer solution with respect thereto and conducting spray-coating thereof and drying. Outer layer-coated particles in Example 16 were prepared by using 800 g of the AQOAT outer layer solution with respect thereto and conducting spray-coating thereof and drying.

Dissolution tests were conducted by using the outer layer-coated particles in Examples 14 to 16 and by employing the same method as in Test Example 1. Results are shown in Table 8. It is to be noted that with regard to the AQOAT which is an enteric film agent, because the film is dissolved in the 2nd fluid (with pH 6.8) in the Dissolution Test but is not dissolved in saliva in the oral cavity, a dissolution test using water in which the AQOAT is not dissolved was additionally implemented.

**[Table 8]**

| | pH of test fluid | Number of paddle revolutions (rpm) | Dissolution rate (%) | | | | |
|---|---|---|---|---|---|---|---|
| | | | 2 minutes | 15 minutes | 30 minutes | 45 minutes | 60 minutes |
| Example 14 | 6.8 | 50 | 7.0 | 77.1 | 85.2 | 88.5 | 91.2 |
| Example 15 | 6.8 | 100 | 36.5 | 84.2 | 91.8 | 94.9 | 96.4 |
| Example 16 | 6.8 | 50 | 60.4 | 97.7 | 98.2 | | |
| Example 16 | water | 50 | 16.1 | 80.1 | 84.7 | 87.7 | 89.9 |

As the results of the dissolution tests, it was found that the tested particles were rapid-release particles in the test fluid with pH 6.8 whose dissolution rates were 80% or more at 15 minutes. On the other hand, with regard to the outer layer-coated particles in Example 15 and Example 16, an unpalatable taste was not felt at two minutes. In addition, with regard to the outer layer-coated particles in Example 14, there was no bitter taste after one minute and the bitter taste was slightly felt after two minutes. However, it was not the bitter taste causing any problems.

### [Test Example 7]

### [Comparative Examples 8 to 9]

An intermediate layer dissolution solution was prepared by dissolving 48 g of TC-5R and 12 g of HPC-L in a mixture solution of 228 g of purified water and 912 g of ethanol. An intermediate layer suspension was prepared by suspending 48 g of TC-5R in a solution in which 12 g of HPC-L was dissolved in 1,140 g of ethanol. An outer layer solution was prepared by dissolving 36 g of ETHOCEL 7 and 4 g of TC-5E in a mixture solution of 36 g of purified water and 324 g of ethanol.

Inputted into a tumbling fluidized bed coating granulating machine (MP-01 model) were 200 g of drug layering particles prepared by employing the same method as in Examples 1 to 6; 1,200 g of the intermediate layer dissolution solution was sprayed thereto for coating, with agitating and fluidizing being conducted; and drying was conducted, thereby obtaining intermediate layer-coated particles. Subsequently, 200 g of the intermediate layer-coated particles were inputted into the tumbling fluidized bed coating granulating machine (MP-01 model); 400 g of the outer layer solution was sprayed thereto for coating, with agitating and fluidizing being conducted; and drying was conducted, thereby obtaining outer layer-coated particles in Comparative Example 8 (which were coated with the outer layer solution whose quantity was equivalent to 20 part by weight of an outer layer coating quantity with respect to 100 part by weight of the intermediate layer-coated particles). In addition, 200 g of the same drug layering particles as mentioned above, 1,200 g of the intermediate layer suspension, and 400 g of the same outer layer solution as mentioned above were used; spray-coating was conducted by employing the same method as in Comparative Example 8 and by means of the tumbling fluidized bed coating granulating machine (MP-01 model); and drying was conducted, thereby obtaining outer layer-coated particles in Comparative Example 9. It is to be noted that for each of Comparative Example 8 and Comparative Example 9, outer layer-coated particles which were coated with the solution whose quantity was equivalent to 10 part by weight of the outer layer coating quantity with respect to 100 part by weight of the intermediate layer-coated particles were sampled halfway, thereby obtaining each sample with a coating quantity of 10%.

Dissolution tests were conducted by using the respective outer layer-coated particles in Comparative Example 8 and Comparative Example 9 and by employing the same method as in Test Example 1. Results are shown in Table 9.

**[Table 9]**

| | Coating quantity of outer layer (%) | Number of paddle revolutions (rpm) | Dissolution rate (%) | | | | |
|---|---|---|---|---|---|---|---|
| | | | 2 minutes | 15 minutes | 30 minutes | 45 minutes | 60 minutes |
| Comparative Example 8 | 10 | 50 | 8.6 | 68.8 | 82.4 | 89.8 | 92.5 |
| | 20 | 50 | 0.8 | 32.5 | 60.1 | 75.0 | 80.6 |
| Comparative Example 9 | 10 | 50 | 10.5 | 61.1 | 79.8 | 87.1 | 91.0 |
| | 20 | 50 | 3.3 | 31.7 | 55.5 | 68.5 | 76.3 |

Next, tastes of the outer layer-coated particles in Comparative Example 8 and Comparative Example 9 were evaluated as in Test Example 1. In other words, four healthy adult men (panelist 1 to panelist 4) took and kept film-coated particles containing the drug whose each quantity is equivalent to 15 mg, in their mouths for two minutes; thereafter, spat out these; and evaluated an unpalatable taste thereof after one minute and after two minutes. Results are shown in Table 10.

**[Table 10]**

| | | Comparative Example 8 | | Comparative Example 9 | |
|---|---|---|---|---|---|
| | | 10% coating | 20% coating | 10% coating | 20% coating |
| 1 minute | Panelist 1 | ± | - | ± | - |
| | Panelist 2 | - | - | - | - |
| | Panelist 3 | - | - | - | - |
| | Panelist 4 | - | - | - | - |
| 2 minutes | Panelist 1 | ± | - | ± | - |
| | Panelist 2 | ± | - | ± | - |
| | Panelist 3 | ± | - | ± | - |
| | Panelist 4 | ± | - | ± | - |

From these results, it was found that there are no differences in dissolution properties and an effect of masking the unpalatable taste between the case where the gelling swelling substance having a viscosity smaller than 10 mPa·s was micronized and suspended to be used as the intermediate layers and the case where the above-mentioned gelling swelling substance was dissolved to be used as the intermediate layers, and in either of the cases, at least one of the dissolution properties or the effect of masking the unpalatable taste become unsuitable.

### [Test Example 8]

An experiment in which a coating quantity of intermediate layers was reduced was implemented by using a gelling swelling substance having a high viscosity.

### [Example 17]

An intermediate layer solution was prepared by suspending 14 g of Ketorol CG in a solution in which 6 g of HPC-L was dissolved in a mixture solution of 11.3 g of purified water and 102 g of ethanol. An outer layer solution was prepared by dissolving 18 g of ETHOCEL 7 and 2 g of TC-5E in a mixture solution of 18 g of purified water and 162 g of ethanol.

Inputted into a tumbling fluidized bed coating granulating machine (MP-01 model) were 200 g of drug layering particles prepared by employing the same method as in Examples 1 to 6; 133.3 g of the intermediate layer solution was sprayed thereto for coating, with agitating and fluidizing being conducted; and drying was conducted, thereby obtaining intermediate layer-coated particles. Subsequently, 200 g of the intermediate layer-coated particles were inputted into the tumbling fluidized bed coating granulating machine (MP-01 model); 200 g of the outer layer solution was sprayed thereto for coating, with agitating and fluidizing being conducted; and drying was conducted, thereby obtaining outer layer-coated particles in Example 17.

A dissolution test was conducted by using the outer layer-coated particles in Example 17 and by employing the same method as in Test Example 1. Because a degree of aggregation of the particles in a dissolution test fluid was high, the number of revolutions of a paddle was set to 100 rpm. Results are shown in Table 11. As a result, although there was a problem in that a degree of mutual adhesion and aggregation among the particles was slightly high, the particles had sufficiently rapid release properties with a dissolution rate of 85% after 30 minutes.

**[Table 11]**

| | Number of paddle revolutions (rpm) | Dissolution rate (%) | | | | |
|---|---|---|---|---|---|---|
| | | 2 minutes | 15 minutes | 30 minutes | 45 minutes | 60 minutes |
| Example 17 | 100 | 10.5 | 67.6 | 85.0 | 90.5 | 93.1 |

The same evaluation of masking properties of an unpalatable taste as in Test Example 1 was implemented. With regard to the outer layer-coated particles in Example 18, there was no bitter taste after one minute and the bitter taste was slightly felt after two minutes, but it was not a bitter taste causing any problems.

### [Test Example 9]

The outer layer-coated particles in Example 10 were used, and orally disintegrating tablets were thereby produced.

### [Example 19]

Particles for tableting prepared by taking and sufficiently mixing 43.85 g of the outer layer-coated particles prepared in Example 10, 30.2 g of D-mannitol (160C), 3.36 g of Mannit Q, 9 g of microcrystalline cellulose (KG-802), 2.7 g of crospovidone (Polyplasdone XL-10), and one g of magnesium stearate were used and were tableted at a tableting pressure of 5 kN, thereby producing tablets each having a diameter of 7.5 mm (flat face non-bevel) and a weight of 150 mg. Each of these tablets disintegrated within 30 seconds when being kept in the oral cavity and gave no feeling of an unpalatable taste causing any problems for approximately two minutes.

As described above, each of the pharmaceutical composition particles according to the present invention includes: the drug-containing core particle; the intermediate layer containing the water-soluble gelling swelling substance and coating the outside of the core particle; and the outer layer containing the water-insoluble substance and coating the outside of the intermediate layer. The gelling swelling substance has the viscosity of 10 mPa·s or more in the 2% aqueous solution at the temperature of 25°C. It is preferable that the content of the water-insoluble substance is greater than or equal to 30% by weight with respect to the total weight of the outer layers, and it is more preferable that the content thereof is greater than or equal to 50% by weight with respect thereto.

In addition, in the pharmaceutical composition particles according to the present invention, it is preferable that the content of the outer layer is greater than or equal to 5% by weight and less than or equal to 50% by weight with respect to the intermediate layer-coated particle having the core particle coated with the intermediate layer.

The embodiment and examples disclosed hereinabove are to be considered in all respects only as illustrative and not restrictive. It is intended that the scope of the invention is, therefore, indicated by the appended claims rather than the foregoing description of the embodiment and examples and that all modifications and variations coming within the meaning and equivalency range of the appended claims are embraced within their scope.

## Claims

1. Pharmaceutical composition particles, each of the pharmaceutical composition particles comprising:
a drug-containing core particle;
an intermediate layer containing a water-soluble gelling swelling substance and coating an outside of the core particle; and
an outer layer containing a water-insoluble substance and coating an outside of the intermediate layer.

2. The pharmaceutical composition particles according to claim 1, wherein the gelling swelling substance has a viscosity of 10 mPa·s or more in a 2% aqueous solution at a temperature of 25°C.

3. The pharmaceutical composition particles according to claim 1 or 2, wherein the outer layer contains the water-insoluble substance whose content is greater than 55% by weight with respect to a total weight of the outer layer.

4. The pharmaceutical composition particles according to any one of claims 1 to 3, wherein a value of swelling power (S) of the gelling swelling substance is greater than or equal to 650.

5. The pharmaceutical composition particles according to any one of claims 1 to 4, wherein a coating quantity of the outer layer is greater than or equal to 5% by weight and less than or equal to 50% by weight with respect to an intermediate layer-coated particle having the core particle coated with the intermediate layer.

6. An orally disintegrating tablet including the pharmaceutical composition particles according to any one of claims 1 to 5.

7. A method for manufacturing pharmaceutical composition particles, comprising:
a pulverization step of pulverizing a gelling swelling substance so as to have an average particle diameter of 15 µm or less;
a suspension step of obtaining a suspension by suspending the gelling swelling substance pulverized at the pulverization step in an organic solvent;
an intermediate layer formation step of forming an intermediate layer on an outside of a core particle by spraying the suspension obtained at the suspension step onto the core particle containing a drug; and
an outer layer formation step of forming an outer layer by coating an outside of the intermediate layer with a water-insoluble substance.

8. The method for manufacturing the pharmaceutical composition particles according to claim 7, wherein the organic solvent is ethanol.
